# EUROPEAN PATENT APPLICATION

(11) **EP 1 955 707 A1**
(43) Date of publication of application: **13.08.2008**
(21) Application number: 06833521.5
(22) Date of filing: 28.11.2006
(51) Int. Cl.: A61K 45/00, A61K 31/195, A61K 31/423, A61P 3/10, A61P 17/00, A61P 25/00

(54) **PHARMACEUTICAL COMPOSITION FOR PREVENTION OR TREATMENT OF NEUROGENIC PAIN**

(30) Priority: 28.11.2005 JP 2005342594
(71) Applicant: Kissei Pharmaceutical Co., Ltd., Matsumoto-shi Nagano 399-8710 (JP)
(72) Inventor: TAKEDA, Hiroo, Azumino-shi 399-8304 (JP); KIGUCHI, Sumiyoshi, Nagano 399-8304 (JP)
(74) Representative: Hayes, Adrian Chetwynd
(86) International application number: PCT/JP2006/323717
(87) International publication number: WO 2007/061114

(57) **Abstract**

The purpose of the present invention is to provide agents useful for the prevention or treatment of a neuropathic pain. That is, the present invention provides agents for the prevention or treatment of neuropathic pain such as painful diabetic neuropathy, postherpetic neuralgia, trigeminal neuralgia, or postoperative or posttraumatic chronic pain or the like, which comprises a β₃ adrenoceptor stimulant as an active ingredient. The present invention also provides a combination of pharmaceuticals comprising a β₃ adrenoceptor stimulant in combination with one or more drugs selected from the group consisting of a psychotropic vitamins, a non-steroidal anti-inflammatory drug, an aldose reductase inhibitor, a lidocaine-like anti-arrhythmic drug, an antidepressant and an anticonvulsant.

## Description

### Field of the invention

The present invention relates to a pharmaceutical composition for the prevention or treatment of neuropathic pain that comprises as an active ingredient a β₃ adrenoceptor (hereinafter referred to as β₃ AR) stimulant.

### Background Art

Neuropathic pain is defined as pain caused or induced when the nervous system is injured temporarily or is in dysfunction. The pain is an intractable algetic disease, which it is resistant to antiphlogistic analgesics and anesthetic analgesics. The typical diseases include cancerous pain, postherpetic neuralgia, trigeminal neuralgia, phantom limb pain, causalgia and painful diabetic neuropathy (or diabetic painful neuropathy) and the like. Among neuropathic pain diseases, there are particularly many patients with painful diabetic neuropathy. It is conjectured that the number of such patients will increase further as that of diabetic patients increases along with changes in life style and aging of the population. Patients with the above-mentioned diseases have pain and sensory abnormality characterized by hyperalgesia and allodynia. It has been reported that because said symptoms persist, patients have deterioration of QOL such as insomnia, loss of appetite or reactive depression (for example, see Non-patent reference 1).

The etiology of neuropathic pain remains mostly unknown. It is conjectured that the disease is induced partially by peripheral and central neuropathy at various levels, and pain is manifested as a metabolic abnormality, regardless of either at peripherally or centrally, blood flow disorder and degeneration of nerve fiber, and changes in synaptic responsiveness lie complexly one upon another.

Neuropathic pain is treated with pharmacotherapy and nerve block therapy. Drugs used in pharmacotherapy include anticonvulsants, psychotropic vitamins, non-steroidal anti-inflammatory drugs, aldose reductase inhibitors, hypoglycemic drugs and lidocaine-like antiarrhythmic drugs and the like. Nerve block therapy includes stellate nerve block, continuous epidural block, nerve root block and the like. However, because hypersusceptibility of neuropathic pain is caused by the breakdown of balance between conduction system and suppression system of pain, these treatments are often insufficiently effective. Therefore, early development of new drugs is expected.

Heretofore, it is not known at all that a β₃ AR stimulant is useful for the treatment of neuropathic pain. Furthermore, there is neither mention nor suggestion thereof in any references.

[Non-patent reference 1] Katsuyuki Moriwaki et al, Pain Clinic, May 2000, Vol. 21, Supplement, pp.S101-S107

### Disclosure of the invention

### Problem that the invention aims to solve

The purpose of the present invention is to provide therapeutic pharmaceuticals for the treatment of neuropathic pain.

### Means to solve the problem

As the result of strenuous research on the above-mentioned problem, the present inventors found, surprisingly, that, a β₃ AR stimulant shows an effect reducing neuropathic pain in diabetic rats induced by streptozotocin (hereinafter referred to as STZ) and Seltzer model rats. In addition, the inventors also found that a β₃ AR stimulant unexpectedly has an excellent effect of the prevention or treatment of neuropathic pain, and thereby forming the basis of the present invention.

That is, the present invention relates to:
[1] a pharmaceutical composition for the prevention or treatment of neuropathic pain, which comprises a β₃ AR stimulant;
[2] a pharmaceutical composition for the prevention or treatment of neuropathic pain, that comprises as an active ingredient a β₃ AR stimulant which is a compound represented by the general formula (I): 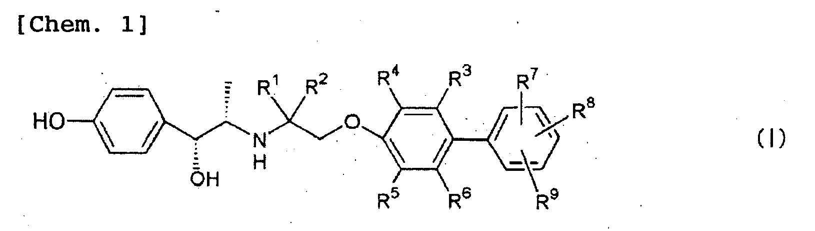 in the formula, R¹ and R² are independently a hydrogen atom or a.lower alkyl group,
   R³, R⁴, R⁵ and R⁶ are independently a hydrogen atom, a halogen atom, a lower alkyl group or a lower alkoxy group,
   R⁷ and R⁸ are independently a hydrogen atom, a halogen atom, a lower alkyl group, a halo(lower alkyl) group, a hydroxy(lower alkyl) group, a cycloalkyl group, a heterocycloalkyl group, a lower alkoxy group, a di(lower alkyl)amino group, a cyclic amino group, a di(lower alkyl)amino(lower alkyl) group, an aryl group, an aryloxy group, an aralkyloxy group, a heteroaryl group, a cyano group, a hydroxy group, a lower acyl group, a lower alkylsulfanyl group, a lower alkylsulfonyl group, a carboxy group, a lower alkoxycarbonyl group or an aralkyloxycarcbonyl group, or in case that R⁷ and R⁸ are adjacent, they bind to each other to form -O-(CH₂)ₘ-O-, -O-(CH₂)ₙ- or -(CH₂)ₚ-,
   wherein m represents an integer from 1 to 3,
   n represents an integer from 2 to 4, and
   p represents an integer from 3 to 5,
   R⁹ is -C(O)-R¹⁰, -A¹-C(O)-R¹⁰, -O-A²-C(O)-R¹⁰ or a tetrazol-5-yl group,
   wherein R¹⁰ represents a hydroxy group, a lower alkoxy group, an aralkyloxy group or -NR¹¹R¹², R¹¹ and R¹² represent independently a hydrogen atom, a lower alkyl group, a carboxy(lower alkyl) group or a lower alkoxycarbonyl(lower alkyl) group, or R¹¹ and R¹² bind together with the nitrogen atom bound to them to form a cyclic amine,
   A¹ is a lower alkylene group or a lower alkenylene group, and
   A² is a lower alkylene group, or a prodrug thereof, or a pharmaceutically acceptable salt thereof;
[3] a pharmaceutical composition as described in the above [1] or [2], wherein the neuropathic pain is painful diabetic neuropathy, postherpetic neuralgia, trigeminal neuralgia, or postoperative or posttraumatic chronic pain;
[4] a pharmaceutical composition as described in any of the above [1] to [3], which is used in combination with one or more drugs selected from the group consisting of a psychotropic vitamins, a non-steroidal anti-inflammatory drug, an aldose reductase inhibitor, a lidocaine-like anti-arrhythmic drug, an antidepressant and an anticonvulsant; and the like.

### Effect of the invention

Pharmaceutical compositions of the present invention extremely increase the nociceptive threshold in models such as STZ-induced diabetic rats and Seltzer model rats, which are the representative models for evaluation of drug efficacy in neuropathic pain, and therefore, are useful for the prevention or treatment of neuropathic pain.

### Brief description of the drawing

[Figure 1]
   Analgesic effects of repeated administration of Compound 3 hydrochloride described below, in STZ-induced diabetic rats are shown. The ordinate in the figure indicates the nociceptive threshold (g), and the abscissa denotes administration groups, wherein Normal indicates a Normal group, Control indicates a Control group, and a numeral denotes a dose of Compound 3 hydrochloride (mg/kg). The symbol "##" means P<0.01 (significant difference from Control group in Aspin-Welch's t test was observed).
[Figure 2]
   Analgesic effects of repeated administration of Compound 1 described below, in STZ-induced diabetic rats are shown. The ordinate in the figure indicates the nociceptive threshold (g), and the abscissa denotes the time (weeks) after STZ administration and the figure indicates the changes of the nociceptive threshold over 10 weeks after STZ administration. Repeated-measures analysis of variance was performed and significant difference of the interaction of group and time was observed between Normal and Control groups (P<0.01). Furthermore, significant difference was observed between Control group and Compound 1 (P<0.05).
[Figure 3]
   Analgesic effects of repeated administration of Compound 2 described below, in STZ-induced diabetic rats are shown. The ordinate in the figure indicates the nociceptive threshold (g), and the abscissa denotes the time (weeks) after STZ administration and figure indicates the changes of the nociceptive threshold over 10 weeks after STZ administration. Repeated-measures analysis of variance was performed and significant difference of the interaction of group and time was observed between Normal and Control groups (P<0.01). Furthermore, significant difference was observed between Control group and Compound 2 (P<0.05).
[Figure 4]
   Analgesic effects of repeated administration of Compound 1, Compound 2 and Compound 3 hydrochloride described below, in Seltzer model rats, are shown. The ordinate in the figure indicates the nociceptive threshold (g), and the abscissa denotes administration groups, wherein Normal indicates a Normal group, Control indicates a Control group, and CBZ indicates carbamazepine. The symbols "#" and "##" means P<0.05 and P<0.01(significant differences from Control group in Aspin-Welch's t test were observed), respectively.

### Best mode to operate the invention

In the present invention, β₃ AR stimulant means a compound having a stimulant action on β₃ receptor, and among them, a compound having a stronger stimulant action on β₃ AR in comparison with that on β₁ AR and β₂ AR is preferable. A compound having a more than 5 times stronger stimulant action on β₃ AR in comparison with that on β₁ AR is more preferable, and moreover, a compound having a more than 10 times stronger stimulant action is further more preferable. In addition, a compound having a more than 5 times stronger stimulant action on β₃ AR in comparison with that on β₂ AR is more preferable, and moreover, a compound having a more than 10 times stronger stimulant action is further more preferable. The each action on β₁ AR, β₂ AR and β₃ AR can be determined by the method described, for example, in International publication No. WO2004/072016 pamphlet.

As β₃ AR stimulants of the present invention, the compounds represented by the above-mentioned general formula (I) can be illustrated.

In another embodiment, as β₃ AR stimulants of the present invention, a compound represented by the following general formula (II), 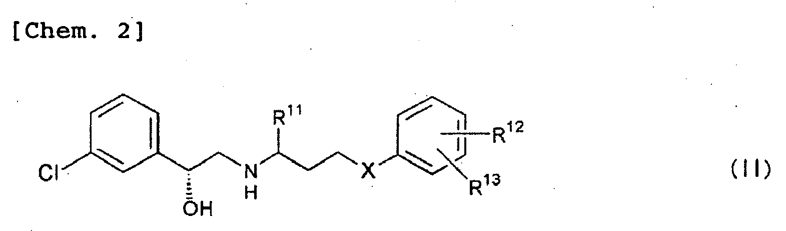 in the formula,
X is a bond, -NH- or -O-;
R¹¹ is a hydrogen atom or a lower alkyl group; and one of R¹² and R¹³ is a hydrogen atom, and the other is -O-Y-COOR¹⁴ (in the formula, R¹⁴ is a hydrogen atom or a lower alkyl group, Y is a bond or -O-) or a group represented by the following general formula (IIa) 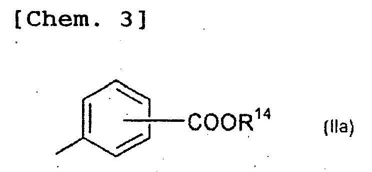 (in the formula, R¹⁴ is a hydrogen atom or a lower alkyl group),
or R¹² and R¹³ form a substituent represented by the following general formula (IIb) 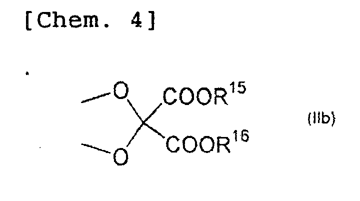 (in the formula, R¹⁵ and R¹⁶ are independently a hydrogen atom or a lower alkyl group), or a substituent represented by the following general formula (IIc) 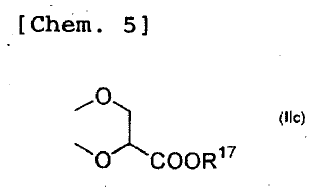 (in the formula, R¹⁷ is a hydrogen atom or a lower alkyl group), or a prodrug thereof, or a pharmaceutically acceptable salt thereof can be illustrated.

In the present invention, the following terms have the following meanings if not otherwise specified especially.
The term "halogen atom" means a fluorine atom, a chlorine atom, a bromine atom or an iodine atom. A fluorine atom or a chlorine atom is preferable.

The term "lower alkyl group" means a straight-chained or branched alkyl group having 1 to 6 carbon atoms. For example, a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a 1-methylbutyl group, a 2-methylbutyl group, a 1,2-dimethylpropyl group, a hexyl group, an isohexyl group or the like can be illustrated. As the lower alkyl group in R¹, R², R³, R⁴, R⁵ and R⁶, an alkyl group having 1 to 4 carbon atoms is preferable, and a methyl group is more preferable. As the lower alkyl group in R⁷, R⁸ and R⁹, an alkyl group having 1 to 4 carbon atoms is preferable, and a methyl group, an ethyl group, a propyl group or an isopropyl group is more preferable.

The term "halo(lower alkyl) group" means a lower alkyl group substituted by the same or different 1 to 3 halogen atoms. For example, a trifluoromethyl group, a 2-chloroethyl group, a 2-fluoroethyl group, a 2,2,2-trifluoroethyl group, a 2,2,2-trichloroethyl group or the like can be illustrated. A trifluoromethyl group is preferable.

The term "hydroxy(lower alkyl) group" means a lower alkyl group substituted by a hydroxy group. For example, a hydroxymethyl group, a 2-hydroxyethyl group, a 1-hydroxyethyl group, a 3-hydroxypropyl group, a 4-hydroxybutyl group or the like can be illustrated. A hydroxymethyl group is preferable.

The term "cycloalkyl group" means a saturated cyclic hydrocarbon group having 3 to 7 carbon atoms. For example, a cyclopropyl group, a cyclobutyl group, a cyclopentyl group, a cyclohexyl group, a cycloheptyl group or the like can be illustrated. A cyclopentyl group or a cyclohexyl group is preferable.

The term "heterocycloalkyl group" means a 3 to 7-membered saturated heterocycle group, which contains an oxygen atom or a sulfur atom in the ring. For example, a tetrahydrofuryl group, a tetrahydrothienyl group, a tetrahydropyranyl group or the like can be illustrated.

The term "lower alkoxy group" means a straight-chained or branched alkoxy group having 1 to 6 carbon atoms. For example, a methoxy group, an ethoxy group, a propoxy group, an isopropoxy group, a butoxy group, an isobutoxy group, a sec-butoxy group, a *tert*-butoxy group, a pentyloxy group, a hexyloxy group or the like can be illustrated. As the lower alkoxy group in R³, R⁴, R⁵ and R⁶, an alkoxy group having 1 to 4 carbon atoms is preferable, and a methoxy group is more preferable. As the lower alkoxy group in R⁷, R⁸ and R⁹, an alkoxy group having 1 to 4 carbon atoms is preferable, and a methoxy group, an ethoxy group, a propoxy group or an isopropoxy group is more preferable. As the lower alkoxy group in R¹⁰, an alkoxy group having 1 to 4 carbon atoms is preferable, and an ethoxy group, a propoxy group, an isopropoxy group or a butoxy group is more preferable.

The term "di(lower alkyl)amino group" means an amino group disubstituted by lower alkyl groups. For example, a dimethylamino group, a diethylamino group or the like can be illustrated.

The term "di(lower alkyl)amino(lower alkyl) group" means a lower alkyl group substituted by a di(lower alkyl)amino group. For example, a dimethlaminomethyl group or the like can be illustrated.

The term "lower acyl group" means a group represented by (lower alkyl)-CO-. For example, an acetyl group, a propionyl group, a butyryl group, an isobutyryl group, a pivaloyl group, a valeryl group, an isovaleryl group or the like can be illustrated. An acetyl group is preferable.

The term "lower alkylsulfanyl group" means a group represented by (lower alkyl)-S-. For example, a methylsulfanyl group, an ethylsulfanyl group, a propylsulfanyl group, an isopropylsulfanyl group, a butylsulfanyl group, a pentylsulfanyl group, a hexylsulfanyl group or the like can be illustrated. A methylsulfanyl group or an ethylsulfanyl group is preferable.

The term "lower alkylsulfonyl group" means a group represented by (lower alkyl)-SO₂-. For example, a methanesulfonyl group, an ethanesulfonyl group, a propanesulfonyl group, a butanesufonyl group, a pentanesulfonyl group, a hexanesulfonyl group or the like ca be illustrated. A methanesulfonyl group is preferable.

The term "lower alkoxycarbonyl group" means a group represented by (lower alkoxy)-CO-. For example, a methoxycarbonyl group, an ethoxycarbonyl group, a propoxycarbonyl group, an isopropoxycarbonyl group, a butoxycarbonyl group, an isobutoxycarbonyl group, a sec-butoxycarbonyl group, a tert-butoxycarbonyl group, a pentyloxycarbonyl group, a hexyloxycarbonyl group or the like can be illustrated. A methoxycarbonyl group, an ethoxycarbonyl group, a propoxycarbonyl group, an isopropoxycarbonyl group or a butoxycarbonyl group is preferable.

The term "aryl group" means an aromatic hydrocarbon group having 6 to 14 carbon atoms, which is unsubstituted or substituted by 1 to 3 groups independently selected from the following group consisting of a halogen atom, a lower alkyl group, a halo(lower alkyl) group, a lower alkoxy group, a hydroxy group, a carboxy group and a lower alkoxycarbonyl group. For example, a phenyl group, a 2-fluorophenyl group, a 3-fluorophenyl group, a 4-fluorophenyl group, a 2-chlorophenyl group, a 3,5-dichlorophenyl group, a 4-methylpheny group, a 4-trifluoromethylphenyl group, a 2-methoxyphenyl group, a 4-methoxyphenyl group, a 4-hydroxyphenyl group, a 4-carboxyphenyl group, a 4-methoxycarbonylphenyl group, a naphthyl group, an anthryl group, a phenanthryl group or the like can be illustrated. A phenyl group is preferable.

The term "aryloxy group" means a group represented by (aryl)-O-. For example, a phenoxy group, a 2-fluorophenoxy group, a 3-fluorophenoxy group, a 4-fluorophenoxy group, a 2-chlorophenoxy group, a 4-chlorophenoxy group, a 3,5-dichlorophenoxy group, a 4-methylphenoxy group, a 4-trifluoromethylphenoxy group, a 2-methoxyphenoxy group, a 4-methoxyphenoxy group, a 2-hydroxyphenoxy group, a 4-carboxyphenoxy group, a 4-methoxycarbonylphenoxy group, a naphthyloxy group, an anthryloxy group, a phenanthryloxy group or the like can be illustrated. A phenoxy group, a 4-fluorophenoxy group, a 4-chlorophenoxy group, a 4-methylphenoxy group or a 4-methoxyphenoxy group is preferable.

The term "aralkyloxy group" means a lower alkoxy group substituted by an aryl group. For example, a benzyloxy group, a phenethyloxy group, a 3-phenylpropyloxy group, a 2-fluorobenzyloxy group, a 3-fluorobenzyloxy group, a 4-fluorobenzyloxy group, a 2-chlorobenzyloxy group, a 3,5-dichlorobenzyloxy group, a 4-methylbenzyloxy group, a 4-trifluoromethylbenzyloxy group, a 2-methoxy-benzyloxy group, a 2-hydroxybenzyloxy group, a 4-carboxy-benzyloxy group, a 4-methoxycarbonylbenzyloxy group or the like can be illustrated. A benzyloxy group is preferable.

The term "aralkyloxycarbonyl group" means a group represented by (aralkyloxy)-CO-. For example, a benzyloxycarbonyl group, a phenethyloxycarbonyl group, a 3-phenylpropyloxycarbonyl group or the like can be illustrated. A benzyloxycarbonyl group is preferable.

The term "heteroaryl group" means a 5 or 6-membered aromatic heterocyclic group which contains 1 to 5 carbon atoms and 1 to 4 hetero atoms independently selected from the group consisting of a nitrogen atom, an oxygen atom and a sulfur atom with the proviso that an adjacent oxygen atom(s) and/or sulfur atom(s) are not contained in the ring. As a heteroaryl group, for example, a pyrrolyl group, a furyl group, a thienyl group, a imidazolyl group, a pyrazolyl group, a 1,2,4-triazolyl group, an oxazolyl group, a thiazolyl group, an isoxazolyl group, a tetrazolyl group, a pyridyl group, a pyrazinyl group, a pyrimidinyl group or the like can be illustrated. All regioisomers of these aromatic heterocyclic groups can be taken into consideration (for example, a 2-pyridyl group, a 3-pyridyl group, a 4-pyridyl group and the like). These heteroaryl groups can be optionally substituted by 1 to 3 groups independently selected from the following group consisting of a halogen atom, a lower alkyl group, a halo(lower alkyl) group, a lower alkoxy group, a hydroxy group, a carboxy group and a lower alkoxycarbonyl group. A preferable heteroaryl group is an imidazolyl group, a pyrazolyl group, a thiazolyl group, a pyridyl group, a pyrazinyl group or a pyrimidyl group.

The term "carboxy(lower alkyl) group" means a lower alkyl group substituted by a carboxy group. For example, a carboxymethyl group, a 2-carboxyethyl group, a 1-carboxyethyl group, a 3-carboxypropyl group, a 4-carboxybutyl group or the like can be illustrated. A carboxymethyl group is preferable.

The term "lower alkoxycarbonyl(lower alkyl) group" means a lower alkyl group substituted by a lower alkoxy carbonyl group. For example, a methoxycarbonylmethyl group, an ethoxycarbonylmethyl group, a propoxycarbonylmethyl group, an isopropoxycarbonylmethyl group, a butoxycarbonylmethyl group, a 2-(ethoxycarbonyl)ethyl group, a 1-(ethoxycarbonyl)ethyl group, a 3-(ethoxycarbonyl)propyl group, a 4-(ethoxycarbonyl)butyl group or the like can be illustrated. A methoxycarbonylmethyl group, an ethoxycarbonylmethyl group, a propoxycarbonylmethyl group, an isopropoxycarbonylmethyl group or a butoxycarbonylmethyl group is preferable.

The term "cyclic amine or cyclic amino group" means a 5 to 7-membered saturated cyclic amino group which can contain an oxygen atom in the ring. For example, a pyrrolidyl group, a piperidyl group, a morpholinyl group or the like can be illustrated.

The term "lower alkylene group" means a straight-chained or branched bivalent saturated hydrocarbon group having 1 to 4 carbon atoms. For example, -CH₂-, -CH₂CH₂-, -CH(CH₃)-, -CH₂CH₂CH₂-, -CH(CH₃)CH₂-,-CH₂CH(CH₃)-, -C(CH₃)₂-,-CH(CH₂CH₃)-, -CH₂CH₂CH₂CH₂- or the like can be illustrated, and -CH₂- is preferable.

The term "lower alkenylene group" means a straight-chained or branched bivalent unsaturated hydrocarbon group having 2 to 4 carbon atoms, and at least a double bond. For example, -CH=CH-, -C(CH₃)=C_{H}-, -CH=CHCH₂-, -CH₂CH=CH-or the like can be illustrated.

In the compound represented by the general formula (I), biphenyl bond means a bond between a phenyl ring binding with R³, R⁴, R⁵ or R⁶ and the other phenyl ring binding with R⁷, R⁸ or R⁹.

In case that a compound represented by the above general formula (I) or (II) has one or more asymmetric carbon atoms, any isomers wherein each asymmetric carbon atom has *R-*configuration or *S-*configuration, and any combination thereof is included in the present invention. In addition, either of a racemic compound, a racemic mixture, a single enantiomer and a diastereomeric mixture thereof is included in the present invention. In case that a compound represented by the above general formula (I) or (II) has one or more geometrical isomers, either of cis-isomer, trans-isomer and a mixture thereof is included in the present invention. Moreover, the compounds represented by the above general formula (I) or (II) include a hydrate thereof or a solvate thereof with a pharmaceutically acceptable solvent such as ethanol or the like.

The compound represented by the general formula (I) or (II) can exist as a salt form. Examples of such salts include acid addition salts with mineral acids such as hydrochloric acid, hydrobromic acid, hydroiodic acid, sulfuric acid, nitric acid, phosphoric acid or the like, acid addition salts with organic acids such as formic acid, acetic acid, methanesulfonic acid, benzenesulfonic acid, p-toluenesulfonic acid, propionic acid, citric acid, succininc acid, tartaric acid, fumaric acid, butyric acid, oxalic acid, malonic acid, maleic acid, lactic acid, malic acid, carbonic acid, glutamic acid, aspartic acid or the like, salts with inorganic bases such as sodium salt, potassium salt, calcium salt or the like, salts with organic bases such as triethylamine, piperidine, morpholine, lysine, ethylenediamine or the like can be illustrated.

In the present invention, the term "prodrug" means a compound which can be converted into a compound represented by the general formula (I) or (II) in the body, and such a prodrug is also within the scope of the present invention. The various forms of prodrug are well known in the present field.

For example, in case that the compound represented by the general formula (I) or (II) has a carboxy group, as a prodrug, an ester which is formed by replacing the hydrogen atom of the aforementioned carboxy group with the following group: a lower alkyl group (for example, a methyl group, an ethyl group, a propyl group, an isopropyl group, a butyl group, a tert-butyl group or the like); a lower acyloxymethyl group (for example, a pivaloyloxymethyl group or the like); a 1-(lower acyloxy)ethyl group (for example, a 1-(pivaloyloxy)ethyl group or the like); a lower alkoxycarbonyloxymethyl group (for example, a tert-butoxycarbonyloxymethyl group or the like); a 1-(lower alkoxycarbonyloxy)ethyl group (for example, a 1-(tert-butoxycarbonyoxy)ethyl group or the like); or a 3-phthalidyl group, can be illustrated.

For example, in case that the compound represented by the general formula (I) or (II) has a hydroxy group, as a prodrug, a compound which is formed by replacing the hydrogen atom of the aforementioned hydroxy group with the following group: a lower acyl group (for example, an acetyl group, a propionyl group, a butyryl group, an isobutyryl group, a pivaloyl group or the like); a lower alkoxycarbonyl group (for example, a methoxycarbonyl group, an ethoxycarbonyl group, a propoxycarbonyl group, an isopropoxycarbonyl group, a tert-butoxycarbonyl group or the like); a succinoyl group; a lower acyloxymethyl group (for example, a pivaloyloxymethyl group or the like); a 1-(lower acyloxy)ethyl group (for example, a 1-(pivaloyloxy)ethyl group or the like); or a lower alkoxycarbonyloxymethyl group (for example, a tert-butoxycarbonyloxymethyl group or the like), can be illustrated.

Additionally, in case that the compound represented by the general formula (I) or (II) has an amino group such as -NH or -NH₂, as a prodrug, a compound which is formed by replacing the hydrogen atom with the following group: a lower acyl group (for example, an acetyl group, a propionyl group, a butyryl group, an isobutyryl group, a pivaloyl group or the like); or a lower alkoxycarbonyl group (for example, a methoxycarbonyl group, an ethoxycarbonyl group, a propoxycarbonyl group, an isopropoxycarbonyl group, a tert-butoxycarbonyl group or the like), can be illustrated.

A prodrug compound can be prepared from a compound represented by the general formula (I) or (II) by the heretofore known methods, for example, in accordance with the methods described in "Protective Groups in Organic Synthesis (third edition)", T. W. Green & P. G. H. Wuts, and references therein.

As preferred examples of β₃ AR stimulants of the present invention, compounds selected from the group consisting of the following compounds can be illustrated.
(R,R)-[4-[2-[[2-(3-Chlorophenyl)-2-hydroxyethyl]-amino]propyl]phenoxyacetic acid sodium salt (hereinafter referred to as Compound 1),
(R,R)-5-[2-[[2-(3-chlorophenyl)-2-hydroxyethyl]-amino]propyl]-1,3-benzoxazol-2,2-dicarbonic acid disodium salt (hereinafter referred to as Compound 2),
4,'-[2-[(1S,2R)-2-hydroxy-2-(4-hydroxyphenyl)-1-methylethylamino]ethoxy]-3-isopropyl-3',5'-dimethyl-biphenyl-4-carboxylic acid (hereinafter referred to as Compound 3),
(R,S)-ethyl-7-[2-[1-(3-chlorophenyl)-1-hydroxyethyl]amino]-1,2,3,4-tetrahydronaph-2-yl-oxyacetic acid,
3'-[[2-[[(2R)-2-(3-chlorophenyl)-2-hydroxyethyl]-amino]ethyl]amino]biphenyl-3-yl-carboxylic acid,
6-[2-(R)-[[2-(R)-(3-chlorophenyl)-2-hydroxyethyl]-amino]propyl]-2,3-dihydro-1,4-benzodioxin-2-(R)-carboxylic acid,
4'-{2-[(1S,2R)-2-hydroxy-2-(4-hydroxyphenyl)-1-methylethylamino]ethoxy}-2,3',5'-trimethylbiphenyl-4-carboxylic acid,
4'-{2-[(1S,2R)-2-hydroxy-2-(4-hydroxyphenyl)-1-methylethylamino]ethoxy}-3-isopropyl-3',5'-dimethylbiphenyl-4-carboxylic acid;
(3-acetyl-4'-{2-[(1S,2R)-2-hydroxy-2-(4-hydroxyphenyl)-1-methylethylamino]ethoxy}-3',5'-dimethylbiphenyl-4-yloxy)acetic acid,
4'-{2-[(1R,2S)-2-hydroxy-2-(4-hydroxyphenyl)-1-methylethylamino]ethoxy}-2,2'-dimethylbiphenyl-4-carboxylic acid,
2-ethyl-4'-{2-[(1S,2R)-2-hydroxy-2-(4-hydroxyphenyl)-1-methylethylamino]ethoxy}-2'-methylbiphenyl-4-carboxylic acid,
4'-{2-[(1S,2R)-2-hydroxy-2-(4-hydroxyphenyl)-1-methylethylamino]ethoxy}-2-isopropyl-2'-methylbiphenyl-4-carboxylic acid,
4'-{2-[(1S,2R)-2-hydroxy-2-(4-hydroxyphenyl)-1-methylethylamino]ethoxy}-2'-methyl-2-propylbiphenyl-4-carboxylic acid,
4'-{2-[(1R,2S)-2-hydroxy-2-(4-hydroxyphenyl)-1-methylethylamino]ethoxy}-2-methoxy-3',5'-dimethylbiphenyl-4-carboxylic acid,
4'-{2-[(1R,2S)-2-hydroxy-2-(4-hydroxyphenyl)-1-methylethylamino]ethoxy}-3',5'-dimethyl-2-propylbiphenyl-4-carboxylic acid,
2-ethyl-4'-{2-[(1R,2S)-2-hydroxy-2-(4-hydroxyphenyl)-1-methylethylamino]ethoxy}-3'-methylbiphenyl-4-carboxylic acid,
4'-{2-[(1R,2S)-2-hydroxy-2-(4-hydroxyphenyl)-1-methylethylamino]ethoxy}-3'-methyl-2-propylbiphenyl-4-carboxylic acid,
3-cyclopentyl-4'-{2-[(1R,2S)-2-hydroxy-2-(4-hydroxy-phenyl)-1-methylethylamino]ethoxy}-3'-methylbiphenyl-4-carboxylic acid,
2-ethyl-3'-fluoro-4'-{2-[(1R,2S)-2-hydroxy-2-(4-hydroxyphenyl)-1-methylethylamino]ethoxy}biphenyl-4-carboxylic acid,
3'-fluoro-4'-{2-[(1R,2S)-2-hydroxy-2-(4-hydroxyphenyl)-1-methylethylamino]ethoxy}-2-isopropylbiphenyl-4-carboxylic acid,
3'-fluoro-4'-{2-[(1R,2S)-2-hydroxy-2-(4-hydroxyphenyl)-1-methylethylamino]ethoxy}-2-propylbiphenyl-4-carboxylic acid,
(4'-{2-[(1S,2R)-2-hydroxy-2-(4-hydroxyphenyl)-1-methylethylamino]ethoxy}-2,3',5'-trimethylbiphenyl-4-yloxy)acetic acid,
3-hydroxy-4'-{2-[(1S,2R)-2-hydroxy-2-(4-hydroxyphenyl)-1-methylethylamino]ethoxy}-3',5'-dimethylbiphenyl-4-carboxylic acid,
4'-{2-[(1R,2S)-2-hydroxy-2-(4-hydroxyphenyl)-1-methylethylamino]ethoxy}-3',5'-dimethyl-3-(p-tolyloxy)-biphenyl-4-carboxylic acid,
3-(4-chlorophenoxy)-4'-{2-[(1R,2S)-2-hydroxy-2-(4-hydroxyphenyl)-1-methylethylamino}ethoxy}-3',5'-dimethylbiphenyl-4-carboxylic acid,
3-(4-fluorophenoxy)-4'-{2-[(1R,2S)-2-hydroxy-2-(4-hydroxyphenyl)-1-methylethylamino]ethoxy}-3',5'-dimethylbiphenyl-4-carboxylic acid,
4'-{2-[(1R,2S)-2-hydroxy-2-(4-hydroxyphenyl)-1-methylethylamino]ethoxy}-3-(4-methoxyphenoxy)-3',5'-dimethylbiphenyl-4-carboxylic acid,
4'-{2-[(1R,2S)-2-hydroxy-2-(4-hydroxyphenyl)-1-methylethylamino]ethoxy}-3'-methyl-3-phenoxybiphenyl-4-carboxylic acid,
4'-{2-[(1R,2S)-2-hydroxy-2-(4-hydroxyphenyl)-1-methylethylamino]ethoxy}-3-(4-methoxyphenoxy)-3'-methylbiphenyl-4-carboxylic acid,
3'-fluoro-4'-{2-[(1R,2S)-2-hydroxy-2-(4-hydroxyphenyl)-1-methylethylamino]ethoxy}-3-(4-methoxyphenoxy)-biphenyl-4-carboxylic acid,
3-(4-chlorophenoxy)-3'-fluoro-4'-{2-[(1R,2S)-2-hydroxy-2-(4-hydroxyphenyl)-1-methylethylamino]ethoxy}-biphenyl-4-carboxylic acid,
4'-{2-[(1R,2S)-2-hydroxy-2-(4-hydroxyphenyl)-1-methylethylamino]ethoxy}-2'-methyl-3-phenoxybiphenyl-4-carboxylic acid,
3-(4-fluorophenoxy)-4'-{2-[(1R,2S)-2-hydroxy-2-(4-hydroxyphenyl)-1-methylethylamino]ethoxy}-2'-methyl-biphenyl-4-carboxylic acid,
4'-{2-[(1R,2S)-2-hydroxy-2-(4-hydroxyphenyl)-1-methylethylamino]ethoxy}-6-methoxy-2'-methylbiphenyl-3-carboxylic acid,
4'-{2-[(1R,2S)-2-hydroxy-2-(4-hydroxyphenyl)-1-methylethylamino]ethoxy}-6-methoxy-3',5'-dimethylbiphenyl-3-carboxylic acid,
6-chloro-4'-{2-[(1R,2S)-2-hydroxy-2-(4-hydroxyphenyl)-1-methylethylamino]ethoxy}-3',5'-dimethyl biphenyl-3-carboxylic acid,
6-chloro-4'-{2-[(1R,2S)-2-hydroxy-2-(4-hydroxy-phenyl)-1-methylethylamino]ethoxy}-3'-methylbiphenyl-3-carboxylic acid,
2-ethyl-4'-{2-[(1R,2S)-2-hydroxy-2-(4-hydroxyphenyl)-1-methylethylamino]ethoxy}biphenyl-4-carboxylic acid,
4'-{2-[(1R,2S)-2-hydroxy-2-(4-hydroxyphenyl)-1-methylethylamino]ethoxy}-2-methylbiphenyl-4-carboxylic acid,
4'-{2-[(1R,2S)-2-hydroxy-2-(4-hydroxyphenyl)-1-methylethylamino]ethoxy}-2-isopropylbiphenyl-4-carboxylic acid,
4'-{2-[(1R,2S)-2-hydroxy-2-(4-hydroxyphenyl)-1-methylethylamino]ethoxy}-2-trifluoromethylbiphenyl-4-carboxylic acid,
(-)-2-[4-(2-{[(1S,2R)-2-hydroxy-2-(4-hydroxyphenyl)-1-methylethyl]amino}ethyl)-2,5-dimethylphenyloxy]acetic acid,
4'-{2-[(1R,2S)-2-hydroxy-2-(4-hydroxyphenyl)-1-methylethylamino]ethoxy}-3-propylbiphenyl-4-carboxylic acid,
4'-{2-[(1R,2S)-2-hydroxy-2-(4-hydroxyphenyl)-1-methylethylamino]ethoxy}-2-propylbiphenyl-4-carboxylic acid,
3-sec-butyl-4'-{2-[(1R,2S)-2-hydroxy-2-(4-hydroxyphenyl)-1-methylethylamino]ethoxy}biphenyl-4-carboxylic acid,
3-cyclopentyl-4'-{2-[(1R,2S)-2-hydroxy-2-(4-hydroxyphenyl)-1-methylethylamino]ethoxy}biphenyl-4-carboxylic acid,
4'-{2-[(1R,2S)-2-hydroxy-2-(4-hydroxyphenyl)-1-methylethylamino]ethoxy}-3-phenoxybiphenyl-4-carboxylic acid,
4'-{2-[(1R,2S)-2-hydroxy-2-(4-hydroxyphenyl)-1-methylethylamino]ethoxy}-3-(4-methoxyphenoxy)biphenyl-4-carboxylic acid,
3-(4-chlorophenoxy)-4'-{2-[(1R,2S)-2-hydroxy-2-(4-hydroxyphenyl)-1-methylethylamino]ethoxy}biphenyl-4-carboxylic acid,
3-(4-fluorophenoxy)-4'-{2-[(1R,2S)-2-hydroxy-2-(4-hydroxyphenyl)-1-methylethylamino]ethoxy}biphenyl-4-carboxylic acid,
4'-{2-[(1R,2S)-2-hydroxy-2-(4-hydroxyphenyl)-1-methylethylamino]ethoxy}-3-(p-tolyloxy)biphenyl-4-carboxylic acid or the like, or a lower alkyl ester thereof, or a pharmaceutically acceptable salt thereof; SR-58611A; KUC-7483; LY-377604; YM-178; GRC-1087; MN-246 and the like. These compounds can be manufactured easily in accordance with methods described in literatures (for example, see)
EP patent publication No. 455006 description,
US patent No. 5106867 description,
International publication No. WO2004/043443 pamphlet,
International publication No. WO00/02846 pamphlet,
International publication No. WO99/31045 pamphlet,
International publication No. WO99/52856 pamphlet or
International publication No. W02004/072016 pamphlet.

The present inventors confirmed that, using β₃ AR stimulants having various structures, they all remarkably exerted increasing effects of the nociceptive threshold in STZ-induced diabetic rats and Seltzer model rats. Therefore, the β₃ AR stimulants of the present invention are extremely useful for the prevention or treatment of neuropathic pain. Neuropathic pain includes, for example, cancerous pain, postherpetic neuralgia, trigeminal neuralgia, phantom limb pain, causalgia, painful diabetic neuropathy or the like.

A pharmaceutical composition of the present invention can be manufactured by admixing or by diluting and dissolving an β₃ AR stimulant with necessary formulation carriers such as fillers, disintegrators, binders, lubricants, diluents, buffers, isotonic agents, antiseptics, humectants, emulsifiers, dispersing agents, stabilizers and solubilizers or the like in various dosage forms in the usual manner.

As administration forms of the pharmaceutical composition of the present invention, forms of oral agent such as powders, granules, fine granules, dry syrup, tablets, capsules or the like can be illustrated, and forms of parenteral agent such as injections, poultices, suppositories or the like can be illustrated. Among them, forms of oral agent are preferable.

A pharmaceutical composition of the present invention can be used occasionally in combination with other drugs that have effects alleviating symptoms of neuropathic pain. As other drugs that have effects alleviating symptoms of neuropathic pain, psychotropic vitamins such as vitamin B₁₂; non-steroidal anti-inflammatory drugs such as indomethacin, diclofenac and the like, aldose reductase inhibitors such as epalrestat, lidocaine-like antiarrhythmic drugs such as mexiletine, lidocaine and the like, antidepressants such as imipramine, amitriptine, mianserin and the like, and anticonvulsants such as carbamazepine, phenytoin and the like, and the like can be illustrated.

### Examples

The present invention is further illustrated in more detail by way of the following Examples. However, the present invention is not limited thereto.

### [Test Example 1] Measurement of the nociceptive threshold in STZ-induced diabetic rats (repeated administration)

STZ (50 mg/kg) was administered intravenously to 7-week old male SD stain rats to induce diabetes mellitus. From the following day of the STZ administration, test article (Compound 3 hydrochloride) was administered orally twice a day, repeatedly (10 rats in each group). Normal and Control groups were administered the medium (0.5% methylcellulose). The nociceptive threshold against pressure stimulation given to the right hind-paw of rats after 2 weeks administration (1 hour after the final administration) of test article was measured by Randall-Selitto method, and compared with Control group.

### [Test Example 2] Measurement of the nociceptive threshold in STZ-induced diabetic rats (repeated administration)

STZ (50 mg/kg) was administered intravenously to male SD stain rats to induce diabetes mellitus. From the following day of the STZ administration, test article (Compound 1 or 2) was administered subcutaneously once a day, repeatedly. Normal and Control groups were administered subcutaneously physiological saline (9-10 rats in each group). Before the STZ administration and every 2 weeks until the 10th week after STZ administration, the nociceptive threshold against pressure stimulation given to the right hind-paw of rats was measured by Randall-Selitto method, and compared with Control group.

### [Test Example 3] Measurement of nociceptive threshold in Seltzer model rats

Fifty-eight, 7-week old male SD rats were anesthetized with pentobarbital (Nembutal injection (a registered trademark)), and the right sciatic nerve was exposed. In the nerve ligation group (48 rats), half of the dorsal portion of the nerve was ligated using 6-0 nylon suture. In Normal group (10 rats), the sciatic nerve was only exposed. From the following day of the model preparation, each test article (Compound 1, Compound 2 or Compound 3 hydrochloride) was repeatedly administered once a day for 2 weeks. Compound 1 (10 rats) and Compound 2 (10 rats) were administered subcutaneously, and Compound 3 hydrochloride (9 rats) and carbamazepine (10 rats) were administered orally. Control, group (9 rats) and Normal group were subcutaneously administered physiological saline. One hour after the final administration of the test articles, the nociceptive threshold against pressure stimulation given to the right hind-paw of rats was measured by Randall-Selitto method, and compared with Control group.

### [Example 1] Analgesic effects of Compound 3 (STZ-induced diabetic rats)

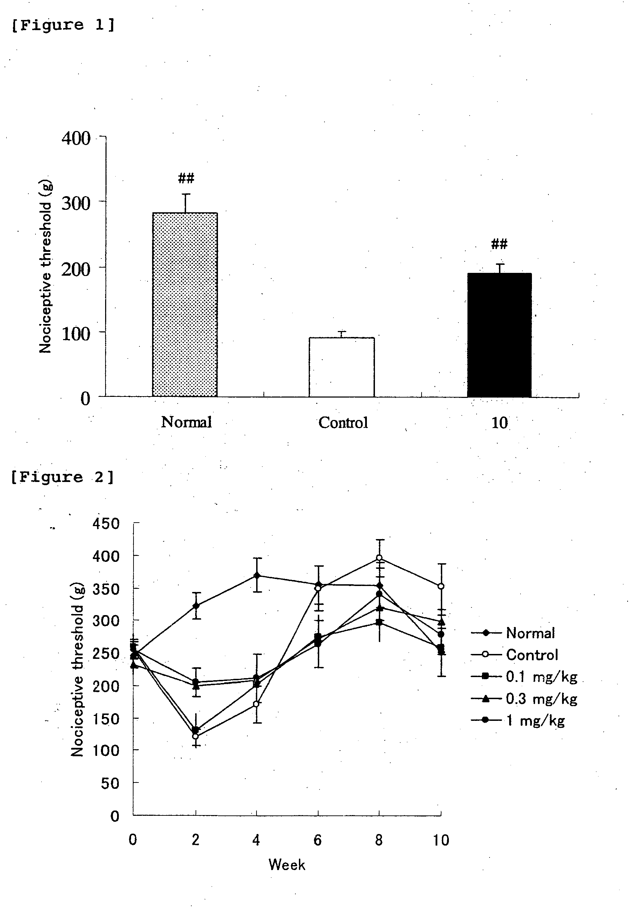

In accordance with the method of Test Example 1, analgesic effect of repeated administration of Compound 3 hydrochloride (10 mg/kg, oral administration) was examined. The nociceptive threshold (mean±SE) at 1 hour after the final administration of the test article is shown in Figure 1.

As a result, the nociceptive threshold was increased remarkably by 14-day repeated administration of Compound 3.

### [Example 2] Analgesic effects of repeated administration of Compound 1 and Compound 2 (STZ-induced diabetic rats)

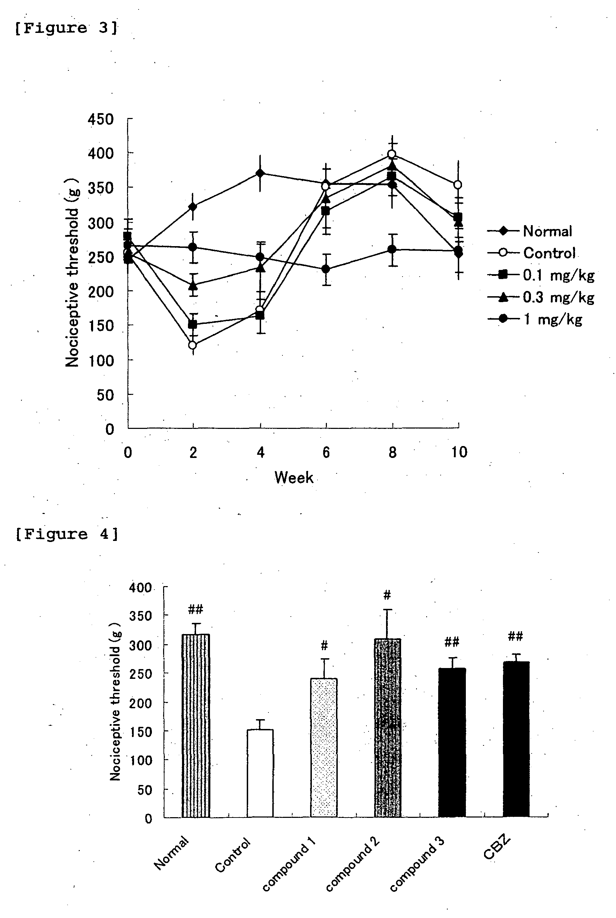

In accordance with the method of Test Example 2, analgesic effects of repeated administration of Compound 1 (0.1, 0.3 and 1 mg/kg, subcutaneous injection) and Compound 2 (0.1, 0.3 and 1 mg/kg, subcutaneous injection) were examined. The changes of the nociceptive thresholds (mean±SE) in each group over 10 weeks after administration of each drug are shown in Figure 2 and Figure 3.

As a result, by 14-day repeated administration of each Compound 1 and Compound 2, the nociceptive threshold was increased clearly in a dose-dependent manner at 0.3 mg/kg or above. Furthermore, the nociceptive thresholds were measured every 2 weeks, and the nociceptive threshold of Control group was decreased in comparison with that of Normal group at the 2nd week, and was increased in comparison with Normal group after the 8th week. At this time, significant difference was observed between Normal and Control groups by repeated-measures analysis of variances. This showed that in this model, hyperalgesia was observed in initial stage, and hypoalgesia by deteriorate of neuropathy was observed in late stage. In groups treated with test articles, the changes of the nociceptive threshold were well controlled small in comparison with Control group. As results of repeated-measures analysis of variances with Control group, Compound 1 and Compound 2 showed the significant differences in comparison with Control group. This indicated that both Compound 1 and Compound 2 have neuroprotective effects.

### [Example 3] Analgesic effects of Compound 1, Compound 2 and Compound 3 (Seltzer model)

In accordance with the method of Test Example 3, analgesic effects of Compound 1 (1 mg/kg, subcutaneous injection), Compound 2 (1 mg/kg, subcutaneous injection), Compound 3 hydrochloride (10 mg/kg, oral administration) and carbamazepine (10 mg/kg, oral administration) were examined. The nociceptive thresholds of before and after treatment (mean±SE) in each group are shown in Figure 4.

As a result, at 2 weeks after model preparation, nociceptive threshold of the right hind paw of the nerve ligation group was significantly lowered in comparison with Normal group. By administration of Compounds 1, 2 and 3 which are β₃ AR stimulants, the nociceptive thresholds of the nerve ligation group were significantly improved in comparison with the preadministration value. This analgesic effect was comparable to that of carbamazepine which is used for trigeminal neuralgia as an analgesic.

Thus, it was confirmed that β₃ AR stimulants having various structures show remarkably increasing effects of nociceptive thresholds in STZ-induced diabetic rats and Seltzer model rats, and furthermore, they have neuroprotective effects. Therefore, β₃ AR stimulants are extremely useful for the prevention or treatment of neuropathic pain.

### Industrial applicability

The pharmaceutical compositions of the present invention are extremely useful as agents for the prevention or treatment of neuropathic pain.

## Claims

1. A pharmaceutical composition for the prevention or treatment of neuropathic pain, which comprises a β₃ adrenoceptor stimulant.

2. A pharmaceutical composition for the prevention or treatment of neuropathic pain, that comprises as an active ingredient a β₃ adrenoceptor stimulant which is a compound represented by the general formula (I): 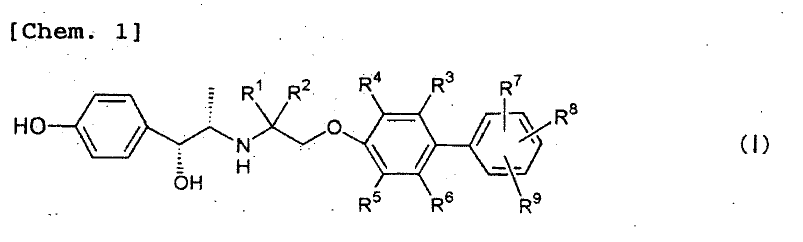 in the formula, R¹ and R² are independently a hydrogen atom or a lower alkyl group,
R³, R⁴, R⁵ and R⁶ are independently a hydrogen atom, a halogen atom, a lower alkyl group or a lower alkoxy group, R⁷ and R⁸ are independently a hydrogen atom, a halogen atom, a lower alkyl group, a halo(lower alkyl) group, a hydroxy(lower alkyl) group, a cycloalkyl group, a heterocycloalkyl group, a lower alkoxy group, a di(lower alkyl)amino group, a cyclic amino group, a di(lower alkyl)amino(lower alkyl) group, an aryl group, an aryloxy group, an aralkyloxy group, a heteroaryl group, a cyano group, a hydroxy group, a lower acyl group, a lower alkylsulfanyl group, a lower alkylsulfonyl group, a carboxy group, a lower alkoxycarbonyl group or an aralkyloxycarcbonyl group, or in case that R⁷ and R⁸ are adjacent, they bind to each other to form -O-(CH₂)ₘ-O-, -O-(CH₂)ₙ- or -(CH₂)ₚ-,
wherein m represents an integer from 1 to 3,
n represents an integer from 2 to 4, and
p represents an integer from 3 to 5,
R⁹ is -C(O)-R¹⁰, -A¹-C(O)-R¹⁰, -O-A²-C(O)-R¹⁰ or a tetrazol-5-yl group,
wherein R¹⁰ represents a hydroxy group, a lower alkoxy group, an aralkyloxy group or -NR¹¹R¹², R¹¹ and R¹² represent independently a hydrogen atom, a lower alkyl group, a carboxy(lower alkyl) group or a lower alkoxycarbonyl(lower alkyl) group, or R¹¹ and R¹² bind together with the nitrogen atom bound to them to form a cyclic amine,
A¹ is a lower alkylene group or a lower alkenylene group, and
A² is a lower alkylene group, or a prodrug thereof, or a pharmaceutically acceptable salt thereof.

3. A pharmaceutical composition as claimed in claim 1 or 2, wherein the neuropathic pain is painful diabetic neuropathy, postherpetic neuralgia, trigeminal neuralgia, or postoperative or posttraumatic chronic pain.

4. A pharmaceutical composition as claimed in any of claims 1 to 3, which is used in combination with one or more drugs selected from the group consisting of a psychotropic vitamins, a non-steroidal anti-inflammatory drug, an aldose reductase inhibitor, a lidocaine-like anti-arrhythmic drug, an antidepressant and an anticonvulsant.
